Europäisches Patentamt

European Patent Office

Office européen des brevets

(19)

(11) Veröffentlichungsnummer : **0 088 154**
**B1**

(12) **EUROPÄISCHE PATENTSCHRIFT**

(45) Veröffentlichungstag der Patentschrift :
16.04.86

(51) Int. Cl.⁴ : **H 04 R 25/00**, A 61 F 11/04,
G 09 B 21/00, H 04 R 11/00,
H 04 R 1/06

(21) Anmeldenummer : 82107910.0

(22) Anmeldetag : 27.08.82

(54) Sprechanbahnungsgerät.

(30) Priorität : 10.03.82 DE 3208678

(43) Veröffentlichungstag der Anmeldung :
14.09.83 Patentblatt 83/37

(45) Bekanntmachung des Hinweises auf die Patenterteilung : 16.04.86 Patentblatt 86/16

(84) Benannte Vertragsstaaten :
AT CH DE FR GB LI NL

(56) Entgegenhaltungen :
DE-A- 2 326 960
DE-A- 2 905 276
FR-A- 1 571 373
US-A- 3 984 708

(73) Patentinhaber : Siemens Aktiengesellschaft
Berlin und München Wittelsbacherplatz 2
D-8000 München 2 (DE)

(72) Erfinder : Pfänder, Wilhelm
Beerenweg 12
D-8802 Sachsen (DE)
Erfinder : Harless, Friedrich
Kleinreuther Weg 40
D-8500 Nürnberg (DE)
Erfinder : Ruckdeschel, Horst
Torstrasse 10
D-8550 Forchheim (DE)
Erfinder : Busch, Dieter
Sportplatzstrasse 9
D-8550 Forchheim (DE)

EP 0 088 154 B1

supported at its edge in an elástic bearing (20) on the housing (14).

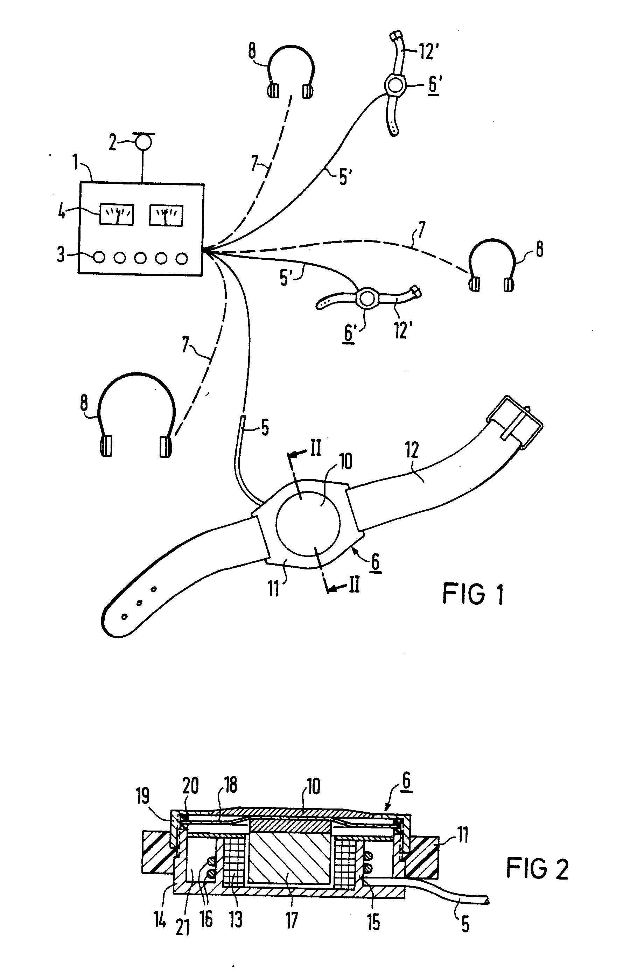

## Revendications

1. Appareil de stimulation de la parole, comportant un transducteur électroacoustique, qui transforme des signaux électriques, qui correspondent à des phénomènes acoustiques, en des vibrations mécaniques pouvant être transmises à la peau, par le fait qu'il contient, dans un boîtier, une bobine dans laquelle pénètre une armature montée élastiquement par rapport à la bobine et réalisée à la manière d'un transmetteur de vibrations sur sa face extérieure et qui est entourée par un circuit magnétique de retour, caractérisé par le fait que l'armature est réunie à un noyau magnétique central (17) de la bobine (13) et qu'un boîtier en forme de pot (14) est réalisé simultanément en tant que circuit magnétique de retour, lequel boîtier (14) entoure, dans la zone du noyau magnétique central (17), une ouverture qui est fermée par une membrane élastique (18) qui porte le noyau magnétique central (17) au niveau de sa face inférieure et un transmetteur de vibrations (10) au niveau de sa face supérieure.

2. Appareil suivant la revendication 1, caractérisé par le fait que le boîtier (14) est monté de façon à pouvoir pivoter autour de son centre, dans un support (11) qui comporte des liaisons aboutissant à un bracelet (12).

3. Appareil suivant la revendication 2, caractérisé par le fait que dans le boîtier (14) se trouve ménagée une rainure (21) dans laquelle le conducteur de raccordement (5) servant à amener les signaux électriques à au moins un enroulement (16) est disposé, d'une manière supprimant la traction et empêchant son arrachement de la bobine (13), autour de la paroi intérieure (15) de la rainure (21) du boîtier (14).

4. Appareil suivant la revendication 1, caractérisé par le fait que le support élastique du noyau (17) est réalisé à l'aide d'une membrane (18) qui est fixée sur le bord d'un organe de support élastique (20) sur le boîtier (14).

FIG 1

FIG 2